(19) Europäisches Patentamt / European Patent Office / Office européen des brevets

(11) **EP 3 873 526 B1**

(12) **EUROPEAN PATENT SPECIFICATION**

(45) Date of publication and mention
of the grant of the patent:
**04.09.2024 Bulletin 2024/36**

(21) Application number: **19809146.4**

(22) Date of filing: **24.10.2019**

(51) International Patent Classification (IPC):
**A61K 39/42** (2006.01)    **A61P 31/14** (2006.01)
**C07K 16/10** (2006.01)    **A61K 39/00** (2006.01)

(52) Cooperative Patent Classification (CPC):
**C07K 16/10; A61K 39/42; A61P 31/14;**
A61K 2039/505; A61K 2039/507; C07K 2317/21;
C07K 2317/33; C07K 2317/34; C07K 2317/76;
C07K 2317/92

(86) International application number:
**PCT/IB2019/059118**

(87) International publication number:
**WO 2020/089742 (07.05.2020 Gazette 2020/19)**

(54) **ANTI-RABIES MONOCLONAL ANTIBODIES AND COCKTAIL THEREOF**

ANTI-RABIES MONOKLONALE ANTIKÖRPER UND COCKTAIL DAVON

ANTICORPS MONOCLONAUX ANTI-RAGE ET COCKTAIL ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **02.11.2018 IN 201821041598**

(43) Date of publication of application:
**08.09.2021 Bulletin 2021/36**

(73) Proprietor: **Zydus Lifesciences Limited
Ahmedabad 382481 (IN)**

(72) Inventors:
• **MENDIRATTA, Sanjeev Kumar**
Ahmedabad, Gujarat 382481 (IN)
• **BANDYOPADHYAY, Sanjay**
Ahmedabad, Gujarat 382481 (IN)
• **KALITA, Pankaj**
Ahmedabad, Gujarat 382481 (IN)
• **KANSAGRA, Kevinkumar**
Ahmedabad, Gujarat 382481 (IN)

(74) Representative: **Hamer, Christopher K. et al
Mathys & Squire
The Shard
32 London Bridge Street
London SE1 9SG (GB)**

(56) References cited:
**WO-A1-2016/078761    WO-A1-2017/027805
WO-A2-2013/048130**

• **NITHYA J GOGTAY ET AL: "Comparison of a
Novel Human Rabies Monoclonal Antibody to
Human Rabies Immunoglobulin for
Postexposure Prophylaxis: A Phase 2/3,
Randomized, Single-Blind, Noninferiority,
Controlled Study", CLINICAL INFECTIOUS
DISEASES, vol. 66, no. 3, 15 September 2017
(2017-09-15), US, pages 387 - 395, XP055655559,
ISSN: 1058-4838, DOI: 10.1093/cid/cix791**
• **BAKKER A B H ET AL: "First administration to
humans of a monoclonal antibody cocktail
against rabies virus: Safety, tolerability, and
neutralizing activity", VACCINE, ELSEVIER,
AMSTERDAM, NL, vol. 26, no. 47, 5 November
2008 (2008-11-05), pages 5922 - 5927,
XP025613275, ISSN: 0264-410X, [retrieved on
20080917], DOI: 10.1016/J.VACCINE.2008.08.050**

- **DATABASE BIOSIS [online] BIOSCIENCES INFORMATION SERVICE, PHILADELPHIA, PA, US; 1 November 2021 (2021-11-01), KANSAGRA KEVINKUMAR ET AL: "A Phase 3, Randomized, Open-label, Noninferiority Trial Evaluating Anti-Rabies Monoclonal Antibody Cocktail (Twinrab (TM)) Against Human Rabies Immunoglobulin (HRIG)", Database accession no. PREV202200044886**
- **CLINICAL INFECTIOUS DISEASES, vol. 73, no. 9, 1 November 2021 (2021-11-01), pages E2722 - E2728, ISSN: 1058-4838(print), DOI: 10.1093/CID/CIAA779**

Remarks:

    The complete document including Reference Table(s) and the Sequence Listing(s) can be downloaded from the EPO website

**Description**

**FIELD OF THE INVENTION**

The present invention relates to a monoclonal antibody capable of binding and neutralizing rabies or rabies related viruses. More specifically, it relates to a cocktail of two monoclonal antibodies capable of binding and neutralizing rabies or rabies related viruses.

**BACKGROUND OF THE INVENTION**

Rabies is a zoonotic disease caused by rabies virus (RABV), the type member of the Lyssavirus genus, and is responsible for >55 000 deaths per annum largely in the developing world, where transmission usually occurs following the bite of an infected dog. If left untreated, the virus progressively infects surrounding neurons and propagates in the central nervous system leading, almost invariably, to death. The disease can be prevented by post-exposure prophylaxis (PEP), which consists of administration of inactivated RABV vaccine together with passive antibody therapy. In a standard passive antibody therapy, polyclonal rabies immunoglobulins (RIG), derived either from immunized human (HRIG) or equine (ERIG) sources, is infused into the wound site. (The Journal of Infectious Diseases 2014; 210:200-8)

Given that the rabies or rabies related viruses cannot be neutralized after it has entered the peripheral nerves at the site of the bite, all post-bite prophylaxis activities that work by neutralizing the virus have to be done at the bite site only. RIGs have been used to neutralize the virus in the wound before the virus enters the nerve. It is obvious that having more amount of drug available at the bite site will provide a better chance of protection of the patient.

As described above, a standard post-bite prophylaxis regimen after a rabid animal bite involves giving of both RIGs and vaccine. RIGs provide early immunity against the virus in a passive manner while vaccine provides later immunity against the virus in an active manner. Any successful regimen has to maintain the right balance of i) RIGs which provide high anti-rabies virus protection in the early phase i.e., immediately (72 hours) after the animal bite whilst ii) it should not neutralize the anti-rabies vaccine (which is also given immediately after the animal bite) and thus developing anti-rabies virus protection via active immunity in the patient in the late phase *i.e.,* up to day 42 or longer.

Clinical trial data of ERIGs, which are horse derived polyclonal anti-rabies sera obtained from repeatedly immunized animals, show that these drugs can be given up to 40 IU/kg in humans. On the other hand, HRIGs which are human donor derived polyclonal anti-rabies sera obtained from immunized human donors, cannot be given beyond 20 IU/kg as a higher dose ends up neutralizing the vaccine thereby failing the post-bite prophylaxis regimen itself.

(Bull. Wld Hlth Org., 1971 45, 303-315)

An ideal regimen should provide higher amounts of RIG at the bite site to provide early phase protection while still allowing the vaccine to provide late phase protection. The difference in doses between the human and horse RIGs is that the human patient can easily recognize the horse derived RIGs as a foreign entity and thereby raising an immune response against the RIG itself. Thus, high amount of ERIG drug becomes available at the bite site and does not remain in the body long enough and allowing the vaccine to provide late phase protection. On the other hand, the human derived RIGs are not recognized as foreign entity and therefore the patient's immune system does not react to it, allowing the HRIG to circulate in patient for longer period of time and neutralizing the vaccine if it is given beyond 20 IU/kg. A higher dose of 40 IU/kg of an HRIG failed the regimen itself.

Additionally, most likely due to similar reason, a recently approved human monoclonal antibody for the post-bite prophylaxis regimen of rabies has been approved to be used at 3.33 IU/kg. (Product insert of Rabishield®).

Therefore, there is a need of an antibody with a low circulating half-life such as a non-human or non-humanized antibody capable of binding and neutralizing the rabies or rabies related viruses as a part of a post-bite prophylaxis regimen for rabies.

Further, these serum derived antibodies often suffer from various drawbacks, including limited availability, batch-to-batch variation, high cost, contamination with blood-borne adventitious agents, and/or risk of adverse reactions. For these reasons, the World Health Organization (WHO) encourages the development and evaluation of alternative biologies for RIG replacement. One such alternative strategy is offered by monoclonal antibodies (mAbs) that are capable of neutralizing a wide range of RABV isolates. Both RIGs and monoclonals are directed against the viral G protein, as several studies have demonstrated that anti-G protein antibodies provide protection against rabies or rabies related viruses. G protein consists of three distinct antigenic sites. Antigenic site I, II and III exist on the rabies virus glycoprotein which have been determined based on the antigenic analysis of glycoprotein variants of CVS-11 virus using 12 neutralizing monoclonal antibodies. (J, gen. Virol. (1983), 64, 843-851). The rabies virus glycoprotein (G) forms surface projections through the viral lipid envelope and is the only protein capable of inducing and reacting with virus-neutralizing antibody (VNA). Studies have established that the isolated G is capable of protecting animals against rabies (J. gen. Virol. (1983), 64, 1649-1656; Proc. Natl. Acad. Sci. USA. Vol. 81, 7194-7198, November 1984). The antigenic sites on the glycoprotein has been studied by using mutants resistant to neutralization by monoclonal antibodies. Three major sites - Site I, II and III has been described in this literature.

RIGs by design comprise of anti-G antibodies directed against multiple epitopes on the G-protein. A monoclonal antibody can bind to only one single epitope on the G-protein. A passive immunity providing product that comprises of only one monoclonal antibody suffers from the risk of a mutant rabies virus escaping the drug if the epitope in question has been lost due to the mutation. Therefore a cocktail of two monoclonal antibodies directed to two distinct non-overlapping epitopes on the G-protein would provide better protection against mutants.

WO2013048130 discloses an anti-rabies antibody which binds rabies virus. The said patent application discloses and teaches chimeric, humanized or human monoclonal antibodies. Such antibodies may not be given in high dose to provide high anti-rabies virus protection in the early phase due to reasons discussed herein above related to human origin RIGs.

WO2016078761 also discloses an anti-rabies antibody which neutralizes both RABV and non-RABV lyssaviruses. The document discloses antigenic sites to which the antibodies and antigen binding fragments bind, which can be used in the diagnosis, prophylaxis, and treatment of RABV infection. An example in the document describes using the antibody for post-exposure prophylaxis and the best combination of antibodies demonstrated protection of 75% of the animals when used with the rabies vaccine.

Accordingly, there is an urgent need for the development of a monoclonal antibody preparation for treatment of rabies which can be produced and supplied in large amounts by cultural synthesis, and can have uniform quality. Such a product would be produced from well characterized cell banks and therefore free from the risk of viral infections and infections from other adventitious agents. Being a monoclonal antibody based product the absolute amount of protein given to the patient as a drug would be much lower than a polyclonal RIG based drug and therefore carry lesser risk on anaphylactic reactions.

Therefore, the current invention provides as described herein, a preparation of monoclonal antibodies capable of binding at two separate sites and neutralizing rabies or rabies related viruses and further a cocktail of such preparation comprising the said two monoclonal antibodies mixed with equipotent amounts for neutralizing rabies or rabies related viruses.

## SUMMARY OF THE INVENTION

The present invention provides a cocktail of two monoclonal antibodies capable of binding and neutralizing rabies or rabies related viruses.

According to the present invention, the said cocktail can neutralize the rabies and rabies related virus that is derived from species such as bats, dogs, cows, mongooses, skunks, and wolves, and thus can be useful in treating a patient that has potentially been infected by the rabies or rabies related viruses derived from a wide variety of species. Further, current invention provides combination of the cocktail of two monoclonal antibodies and anti-rabies vaccine for use in Post exposure prophylaxis (PEP) of exposure with rabies or rabies-related viruses.

## BRIEF DESCRIPTION OF FIGURE

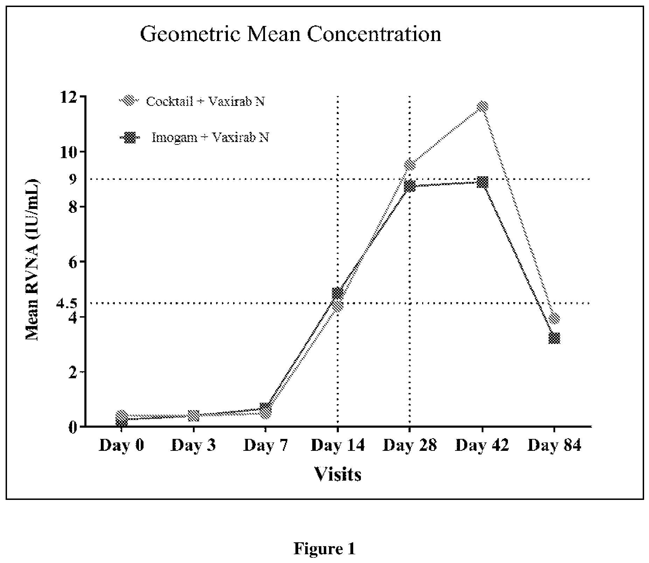

Figure 1 shows linear plot of mean RVNA concentration versus time curves after administration of cocktail of mAb A and mAb B + vaccine and Imogam® + vaccine to patients with suspected rabid animal bite. Cocktail was delivered at a dose of 40 IU/kg while Imogam® (HRIG) was given at its approved dose of 20 IU/kg. After day 14, the cocktail of the present invention maintains higher mean RVNA titers in comparison to HRIG.

### List of abbreviations used herein the specification:

Abbreviations of amino acids as used in the current application are provided in below table:

| Full Name | Abbreviation (3 Letter) | Abbreviation (1 Letter) |
| --- | --- | --- |
| Alanine | Ala | A |
| Arginine | Arg | R |
| Asparagine | Asn | N |
| Aspartate | Asp | D |
| Cysteine | Cys | C |

(continued)

| Full Name | Abbreviation (3 Letter) | Abbreviation (1 Letter) |
|---|---|---|
| Glutamate | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Other abbreviations used in the present application:

ABV: Australian bat virus
BHK: Baby Hamster Kidney fibroblasts
CDRH1: Complementarity determining region 1 of heavy chain
CDRH2: Complementarity determining region 2 of heavy chain
CDRH3: Complementarity determining region 3 of heavy chain
CDRL1: Complementarity determining region 1 of light chain
CDRL2: Complementarity determining region 2 of light chain
CDRL3: Complementarity determining region 3 of light chain
CHO: Chinese hamster ovary
CSO: Cynoglossus Semilaevis Ovary
CVS: Challenge Virus Strain
DP: Drug product
DS: Drug substance
EBLV: European bat lyssavirus
ERIGs: Equine rabies immunoglobulins
FAVN: Fluorescent antibody virus neutralisation
FFU: Fluorescence-Forming Unit
FITC: Fluorescein isothiocyanate
HC: Heavy chain
HCVR: Heavy chain variable region
HEK: Human embryonic kidney
HeLa: Henrietta Lacks
HRIGs: Human rabies immunoglobulins
LC: Light chain
LCVR: Light chain variable region
mAb A: Monoclonal antibody A
mAb B: Monoclonal antibody B
MCB: Master cell bank
NA cells: Neuroblastoma cells
PBS: Phosphate buffer saline

PEP: Post exposure prophylaxis
RABV: Rabies virus
RFFIT: Rapid fluorescent focus inhibition test
RIGs: Rabies immunoglobulins
RVNA: Rabies virus neutralizing antibody
SRIG: Standard rabies immunoglobulin
SV: Street Virus
WCB: Working cell bank

**LIST OF SEQUENCE LISTINGS USED HEREIN THE SPECIFICATION:**

SEQ ID NO. 1: SSWMH (CDRH1 of mAb A)
SEQ ID NO. 2: QTHPNSGYTNYNEKFKG (CDRH2 of mAb A)
SEQ ID NO. 3: ESGDGPHWYFDV (CDRH3 of mAb A)
SEQ ID NO. 4: KASQDVSTAVA (CDRL1 of mAb A)
SEQ ID NO. 5: SASYRYT (CDRL2 of mAb A)
SEQ ID NO. 6: QQHYSSPHT (CDRL3 of mAb A)
SEQ ID NO. 7:

QVQLQQPGSVLVRPGASVKLSCKTSGYAFTSSWMHWAKQRPGQGLEWIGQTHPNS
GYTNYNEKFKGKATLTVDTSSSTAYVDLSSLTSEDSAVYYCARESGDGPHWYFDV
WGAGTAVTVSS (HCVR of mAb A)

SEQ ID NO. 8:

DIVMTQSHKFMSTSVGDRVSITCKASQDVSTAVAWYQQKPGQSPKLLIYSASYRYT
GVPDRFTGSGSGTDFTFTISSVQAEDLAVYYCQQHYSSPHTFGGGTKLETK (LCVR of
mAb A)

SEQ ID NO. 9:

QVQLQQPGSVLVRPGASVKLSCKTSGYAFTSSWMHWAKQRPGQGLEWIGQTHPNS
GYTNYNEKFKGKATLTVDTSSSTAYVDLSSLTSEDSAVYYCARESGDGPHWYFDV
WGAGTAVTVSSAKTTPPSVYPLAPGSAAQTNSMVTLGCLVKGYFPEPVTVTWNSGS
LSSGVHTFPAVLQSDLYTLSSSVTVPSSTWPSETVTCNVAHPASSTKVDKKIVPRDCG
CKPCICTVPEVSSVFIFPPKPKDVLTITLTPKVTCVVVDISKDDPEVQFSWFVDDVEVH
TAQTQPREEQFNSTFRSVSELPIMHQDWLNGKEFKCRVNSAAFPAPIEKTISKTKGRP
KAPQVYTIPPPKEQMAKDKVSLTCMITDFFPEDITVEWQWNGQPAENYKNTQPIMD
TDGSYFVYSKLNVQKSNWEAGNTFTCSVLHEGLHNHHTEKSLSHSPGK    (HC of
mAb A)

SEQ ID NO. 10:

DIVMTQSHKFMSTSVGDRVSITCKASQDVSTAVAWYQQKPGQSPKLLIYSASYRYT GVPDRFTGSGSGTDFTFTISSVQAEDLAVYYCQQHYSSPHTFGGGTKLETKRADAAP TVSIFPPSSEQLTSGGASVVCFLNNFYPKDINVKWKIDGSERQNGVLNSWTDQDSKD STYSMSSTLTLTKDEYERHNSYTCEATHKTSTSPIVKSFNRNEC (LC of mAb A)

SEQ ID NO. 11:

QVQLKESGPGLLAPSQSLSITCTVSGFSLTGHGVNWVRQPPGKGLEWLGIIWADGTT NYNSALKSRLSISKDNSKSQVFLKMNSLQTDDTASYYCAREGDISGYYFDYWGQGT TLTVSSAKTTPPSVYPLAPGCGDTTGSSVTLGCLVKGYFPESVTVTWNSGSLSSSVHT FPALLQSGLYTMSSSVTVPSSTWPSQTVTCSVAHPASSTTVDKKLEPSGPISTINPCPP CKECHKCPAPNLEGGPSVFIFPPNIKDVLMISLTPKVTCVVVDVSEDDPDVQISWFVN NVEVHTAQTQTHREDYNSTIRVVSTLPIQHQDWMSGKEFKCKVNNKDLPSPIERTIS KIKGLVRAPQVYILPPPAEQLSRKDVSLTCLVVGFNPGDISVEWTSNGHTEENYKDT APVLDSDGSYFIYSKLNMKTSKWEKTDSFSCNVRHEGLKNYYLKKTISRSPGK     (HC of mAb B)

SEQ ID NO. 12:

DVQMTQTTSSLSASLGDRVTITCRPSQDINNYLSWYQQKPDGTVKLLIYYTSRLHSG VPSRFSGSGSGTDYSLTISNLEQEDFATYFCQQGNTLPPTFGGGTKLEIKRADAAPTV SIFPPSSEQLTSGGASVVCFLNNFYPKDINVKWKIDGSERQNGVLNSWTDQDSKDST YSMSSTLTLTKDEYERHNSYTCEATHKTSTSPIVKSFNRNEC (LC of mAb B)

**DEFINITIONS**

The term "cocktail" as used herein is a mixture of at least two monoclonal antibodies as two independent active drug substances. The cocktail according to the present invention comprises preferably mixture of mAb A and mAb B. Preferably, the said cocktail is prepared in the formulation buffer. Cocktail is prepared by mixing therapeutic amounts, preferably equipotent amounts of the two active drug substance materials of monoclonal antibodies in the formulation buffer. Finally, it is formulated with other suitable pharmaceutical excipient(s) to prepare a stable pharmaceutical composition of anti-rabies monoclonal antibodies which can be used for therapeutic or treatment purpose.

The term "PEP" or "post exposure prophylaxis" as used herein includes following steps:

1. All bite wounds, scratches and RABV-exposure sites should be attended to as soon as possible after the exposure; thorough washing and flushing of the wound for approximately 15 minutes, with soap or detergent and copious amounts of water, is required. Where available, an iodine-containing, or similarly viricidal, topical preparation should be applied to the wound.
2. A series of rabies vaccine injections should be administered promptly after an exposure as a part of the active immunization component of the PEP.
3. RIG should be administered for severe category III exposures as a part of the passive immunization component of the PEP.

Rabies vaccine according to the present invention may include any anti-rabies vaccine known in the art. RIG according to the present invention may include murine monoclonal antibody or cocktail of at least two monoclonal antibodies.

The term "mAb A" as used herein is a monoclonal antibody which can bind rabies or rabies related viruses and neutralize it. A monoclonal antibody mAb A according to the current invention comprising a CDRH1 comprising a polypeptide set forth in SEQ ID NO: 1, a CDRH2 region comprising a polypeptide set forth in SEQ ID NO: 2, a CDRH3 region comprising a polypeptide set forth in SEQ ID NO: 3, a CDRL1 comprising a polypeptide set forth in SEQ ID NO: 4, a CDRL2 region comprising a polypeptide set forth in SEQ ID NO: 5 and a CDRL3 region comprising a polypeptide set forth in SEQ ID NO: 6. HCVR and LCVR region of mAb A according to the current invention is a polypeptide set forth in SEQ ID NO: 7 and a polypeptide set forth in SEQ ID NO: 8, respectively. Further, heavy chain and light chain of mAb A according to the current invention is a polypeptide set forth in SEQ ID NO: 9 and a polypeptide set forth in SEQ ID NO: 10, respectively. Preferably, mAb A according to the present invention is a murine monoclonal antibody. In one of aspects, the current invention provides mAb A comprising IgG1 constant region, preferably murine IgG1. It binds to site II on G protein of rabies virus envelope.

The term "mAb B" as used herein is a monoclonal antibody which can bind rabies or rabies related viruses and neutralize it. A monoclonal antibody mAb B according to the current invention comprising heavy chain and light chain of mAb B according to the current invention is a polypeptide set forth in SEQ ID NO: 11 and a polypeptide set forth in SEQ ID NO: 12, respectively. Preferably, mAb B according to the present invention is a murine monoclonal antibody. In one of aspects, the current invention provides mAb B comprising IgG2b constant region, preferably murine IgG2b. It binds to site III on G protein of rabies virus envelope.

The term "antibody" as referred to herein includes whole antibodies and any antigen-binding fragment (i.e., "antigen-binding portion") or single chains thereof. An "antibody" refers to a glycoprotein comprising at least two heavy (H) chains and two light (L) chains interconnected by disulfide bonds, or an antigen-binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. In a preferred aspects, the antibody according to the present invention is a murine antibody.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "pharmaceutical composition" refers to preparations which are in such form as to permit the biological activity of the active ingredients to be unequivocally effective. The term "pharmaceutical formulation" or "pharmaceutical composition" or "composition" can be used here interchangeably. "Pharmaceutically acceptable" carrier or excipients are those which can reasonably be administered to a subject mammal to provide an effective dose of the active ingredient employed.

The term "excipient" refers to an agent that may be added to a formulation to stabilize the active drug substance in the formulated form to adjust and maintain osmolality and pH of the pharmaceutical preparations. Examples of commonly used excipients include, but are not limited to, sugars, polyols, amino acids, surfactants, and polymers. "Pharmaceutically acceptable" excipients are those which can reasonably be administered to a subject mammal to provide an effective dose of the active ingredient employed.

In a pharmacological sense, in the context of the present invention, a "therapeutic amount" or "effective amount" of an antibody refers to an amount effective in the prevention or treatment of a disorder for the treatment of which the antibody is effective.

The term "equipotent amount" according to the present invention refers to an amount of each antibody which provides same potency with respect to another antibody or antibodies present in cocktail preparation. Potency of an antibody according to the current invention can be measured in international unit. The potency of the rabies or rabies related viruses neutralizing antibodies is determined by RFFIT method in comparison with an 'Anti-rabies Immunoglobulin, Human WHO International Standard' (RAI) or a validated internal reference standard against the said international standard.

The terms "patient" and "subject" are used interchangeably and are used in their conventional sense to refer to a living organism suffering from or prone to a condition that can be prevented or treated by administration of a composition of the present invention, and includes animals. The term "Animal" refers to a human or non-human animal, including, but not limited to, farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese and non-human primates, including, but not limited to, monkeys, chimpanzees and other apes and monkey species. The term does not denote a particular age. Thus, adult, juvenile and newborn individuals are of interest.

The term "rabies virus" as used herein include rabies and rabies related viruses. According to the present invention, murine antibodies which can bind to site II or site III of G protein of any virus can be used for the treatment in which activity of said virus is detrimental.

The term "exposure" as used herein includes bites, scratches and licks by a rabid animal.

The term "Imogam®" as used herein is an approved HRIG available in the market. It is known to the person skilled in the art.

EMBODIMENTS OF THE INVENTION

In one embodiment, the present invention provides a cocktail of two monoclonal antibodies comprising one monoclonal antibody having heavy chain and light chain comprising polypeptides set forth in SEQ IDs NO.9 and 10, respectively, and second monoclonal antibody having heavy chain and light chain comprising polypeptides set forth in SEQ IDs NO. 11 and 12, respectively. The current invention provides a cocktail of two monoclonal antibodies capable of binding and neutralizing rabies or rabies related viruses.

In a preferred embodiment, the present invention provides cocktail of two murine monoclonal antibodies for the PEP of exposure with rabies and rabies-related viruses.

In one of the embodiments, the present invention provides cocktail of two murine monoclonal antibodies which can be used at higher dose than the approved dose of HRIG or human monoclonal antibodies or humanized monoclonal antibodies. In a preferred embodiment, the present invention provides cocktail of two murine monoclonal antibodies which can be used at a dose more than 20 IU/kg. In a more preferred embodiment, the present invention provides cocktail of two murine monoclonal antibodies which can be used at a dose between 20 IU/kg to 100 IU/kg.

In another embodiment, the present invention provides cocktail of two murine monoclonal antibodies for the PEP of exposure with rabies and rabies-related viruses wherein the cocktail allows the active immunization to generate and maintain an effective immune response.

In a preferred embodiment, the present invention provides combination of cocktail of two murine monoclonal antibody and anti-rabies vaccine for the PEP of exposure with rabies and rabies-related viruses.

In preferred embodiment, the present invention provides use of cocktail of two monoclonal antibodies for the PEP of exposure with rabies and rabies-related viruses.

In a preferred embodiment, the current invention provides a composition comprising cocktail of two monoclonal antibodies capable of binding and neutralizing rabies or rabies related viruses. In another embodiment, the composition according to the present invention comprises cocktail of two monoclonal antibodies and pharmaceutical excipients.

In one of the embodiments, the current invention provides a process of making antibody as claimed in any preceding claim by cell culture method comprising:

i) culturing the host cell expressing monoclonal antibody capable of binding and neutralizing rabies or rabies related viruses; and
ii) isolating and recovering monoclonal antibody capable of binding and neutralizing rabies or rabies related viruses expressed in the said host cell.

In a preferred embodiment, the present invention provides a kit comprising cocktail of two monoclonal antibodies capable of binding and neutralizing rabies or rabies related viruses.

In one of the embodiments, present invention provides a method for diagnosing rabies using the cocktail of at least two monoclonal antibodies capable of binding and neutralizing rabies or rabies related viruses.

In one of the embodiment, the present invention is to provide a method of detecting rabies or rabies related viruses using the cocktail of at least two monoclonal antibodies capable of binding and neutralizing rabies or rabies related viruses.

DETAILED DESCRIPTION OF THE INVENTION

As described herein above, rabies is one of the most dangerous disease for human which causes death if not treated on time. A Post-exposure prophylaxis (PEP) regimen preferably involves two components - one providing active immunity and the other providing passive immunity. The active immunity is provided by a vaccine which works by activating the immune system of the patient/subject and generates immunity against the virus in question in a delayed manner i.e., only on or after seven days of the vaccination. The passive immunity is provided by readymade antibodies, such as polyclonal antibodies (HRIG, ERIG etc.), monoclonal antibodies or their cocktail, which provides anti-virus protection immediately after injection into the patient/subject. Since the passive immunization carries significant risk of neutralizing the active immunization of debilitating the PEP and putting the patient's life at risk, a right balance of dose of passive immunization and type of passive immunization in terms of its circulating half-life in the patient is important in providing sufficient amount and type of neutralizing antibodies through passive immunization that provide protection/immunity in the early phase while allowing the vaccine (active immunization) to carry out its function uninterruptedly together providing an unbroken window of immune protection to the patient. So far passive immunization has largely been provided by conventional products namely, HRIG and ERIG which are used at 20 IU/kg and 40 IU/kg, respectively. HRIG and ERIG as described previously are unsafe because of the potential risk of carrying pathogens. Attempts at developing safer replacement products in the form of monoclonal humanized/human antibodies have led to products that are capable of providing only very low potency of antibodies as early passive immunization because at higher doses they end up neutralizing the active immunization. Thus there is a need of a passive immunization product which is safer than the conventional products and also provides comparable potency.

Present invention provides a cocktail of two murine monoclonal antibodies as the passive immunization component of the PEP of exposure with rabies or rabies related viruses along with a vaccine as active immunization component. A cocktail of two murine monoclonal antibodies according to the present invention can be given at a higher dose than approved dose of HRIG or human monoclonal antibodies or humanized monoclonal antibodies without significantly neutralizing the active immunization provided by an anti-rabies vaccine given as a part of PEP. The cocktail of two murine monoclonal antibodies according to the current invention can be given at a dose more than 20 IU/kg, preferably at a dose between 20 IU/kg to 100 IU/kg. The cocktail of the present invention does not significantly neutralize the active immunization provided by an anti-rabies vaccine given as a part of PEP at a dose more than 20 IU/kg, preferably at a dose between 20 IU/kg to 100 IU/kg. Thus, a cocktail of two murine monoclonal antibodies according to the current invention, for PEP allows the active immunization to generate and maintain an effective immune response against rabies and rabies-related viruses. Vaccine according to the present invention is anti-rabies vaccine known in the art. The present invention provides a cocktail of two murine monoclonal antibodies capable of binding and neutralizing rabies and rabies related viruses for the PEP of exposure with rabies and rabies-related viruses.

Monoclonal antibodies mAb A and mAb B:

A monoclonal antibody capable of binding and neutralizing rabies or rabies related viruses according to the current invention comprises HCVR comprising a CDRH1 comprising a polypeptide set forth in SEQ ID NO: 1, a CDRH2 region comprising a polypeptide set forth in SEQ ID NO: 2, and a CDRH3 region comprising a polypeptide set forth in SEQ ID NO: 3. A monoclonal antibody capable of binding and neutralizing rabies or rabies related viruses according to the current invention comprises LCVR comprising a CDRL1 comprising a polypeptide set forth in SEQ ID NO:4, a CDRL2 region comprising a polypeptide set forth in SEQ ID NO: 5, and a CDRL3 region comprising a polypeptide set forth in SEQ ID NO: 6. A monoclonal antibody capable of binding and neutralizing rabies or rabies related viruses according to the current invention comprises HCVR comprising a polypeptide set forth in SEQ ID NO: 7.

A monoclonal antibody capable of binding and neutralizing rabies or rabies related viruses according to the current invention comprises LCVR comprising a polypeptide set forth in SEQ ID NO: 8.

A monoclonal antibody capable of binding and neutralizing rabies or rabies related viruses according to the current invention comprises heavy chain comprising a polypeptide set forth in SEQ ID NO: 9.A monoclonal antibody capable of binding and neutralizing rabies or rabies related viruses according to the current invention comprises light chain comprising a polypeptide set forth in SEQ ID NO: 10.

A monoclonal antibody comprising polypeptides set forth in SEQ ID NO: 9 and 10 of heavy chain and light chain, respectively is referred herein as mAb A. mAb A comprising amino acid sequences of HCVR and LCVR as set forth in SEQ ID NO.s 7 and 8, respectively. Further, mAb A comprising amino acid sequences of CDRH1, CDRH2 and CDRH3 as set forth in SEQ ID NO.s 1, 2 and 3, respectively. Furthermore, mAb A comprising amino acid sequences of CDRL1, CDRL2 and CDRL3 as set forth in SEQ ID NO.s 4, 5 and 6, respectively.

A monoclonal antibody capable of binding and neutralizing rabies or rabies related viruses according to the current invention comprises heavy chain comprising a polypeptide set forth in SEQ ID NO: 11.

A monoclonal antibody capable of binding and neutralizing rabies or rabies related viruses according to the current invention comprises light chain comprising a polypeptide set forth in SEQ ID NO: 12.

A monoclonal antibody comprising polypeptides set forth in SEQ ID NO: 11 and 12 of heavy chain and light chain, respectively is referred herein as mAb B.

Preferably, mAb A and mAb B are murine antibodies according to the present invention.

Preferably, the present invention provides a cocktail of two monoclonal antibodies i.e. mAb A and mAb B capable of binding and neutralizing rabies or rabies related viruses. Preferably, cocktail comprises equipotent amounts of antibodies capable of binding and neutralizing rabies or rabies related viruses. One of the two antibodies according to the current invention is a monoclonal antibody comprising amino acid sequences of HCVR and LCVR as set forth in SEQ ID NO.s 7 and 8, respectively. Second monoclonal antibody according to the current invention is a monoclonal antibody comprising an amino acid sequences of heavy chain and light chain as set forth in SEQ ID NO.s 11 and 12, respectively.

A cocktail according to the present invention comprising one monoclonal antibody having heavy chain and light chain comprising polypeptides set forth in SEQ ID NO.s 9 and 10, respectively and second monoclonal antibody having heavy chain and light chain comprising a polypeptides set forth in SEQ ID NO.s 11 and 12, respectively.

Monoclonal antibodies for a cocktail preparation are selected based on criteria, such as binding specificity, spectrum of neutralization of lyssaviruses, biological activity, neutralizing potency etc and industrial viability. Various lyssaviruses strains are isolated from specific species across the various geographical areas. For example, RABV (genotype 1) of dogs from Asia (India, Philippines, and Thailand etc.), Africa (North Africa, sub-Saharan Africa, etc.), and the New World and from mongoose(s) from South Africa were included.

Primarily, RABV (genotype 1) of dogs from Asia (India, Philippines, and Thailand etc), Africa (North Africa, sub-Saharan Africa, etc.), and the New World and, from mongoose(s) from South Africa were included. Furthermore, the neutralization capability of the antibodies was also tested against rabies related viruses, namely, EBLV-1, -2, (genotype 5, 6) ABLV (genotype 7), Duvenhage virus (genotype 4), Irkut virus, Khujand and Aravan.

Virus neutralization was undertaken with rabies and rabies related viruses CVS 11, SAD B19, PV, Kelev, EBLV1, EBLV2, ABV, Duvenhage, Irkut, Aravan, and Khujand. Virus neutralization was also undertaken with rabies and rabies related isolates from European Fox, Dog Turkey, Dog Ethiopia, Dog India, Dog Mexico, Wolf Sarajevo, Bobcat-USA, East European fox, Polar fox, Dog Azerbaijan, Dog Nepal, Raccoon, SE US, Gray fox TX, Arctic fox Alaska, Skunk SC US, Skunk CA.

Rapid fluorescent focus inhibition test (RFFIT) or fluorescent antibody virus neutralisation test (FAVN) assays were performed to study neutralization of broad spectrum of lyssaviruses strains as mentioned in examples provided herein below.

The antibodies are produced in an Expression System which may comprise of an Expression Vector and a host cell line. Any expression vector known to the skilled person can be used in the present invention, and the choice of the expression vector is dependent on the nature of the host cell of choice. Introduction of the vector in host cells can be effected by, but not limited to, calcium phosphate transfection, virus infection, DEAE dextran mediated transfection, lipofectamin transfection or electroporation, and skilled person can select and use an introduction method suitable for the expression vector and host cell used. The vector contains all essential elements required to express antibody(s). Preferably, the vector contains one or more selectable markers, but is not limited thereto, and a vector containing no selectable marker may also be used.

The present invention provides a host cell that comprises the expression vector transformed into a host cell to produce the monoclonal antibody of the present invention. In the present invention, the host cell may comprise cells of mammalian, plant, insect, fungal or bacterial origin, but is not limited thereto. A mammalian cell can be selected from, but is not limited thereto, CHO cells, F2N cells, CSO cells, BHK cells, Bowes melanoma cells, HeLa cells, 911 cells, AT1080 cells, A549 cells, HEK 293 cells and HEK293T cells. Suitable mammalian host cell known to skilled person can be used for the development of antibodies and their cocktail of the present invention. Preferably, host cell used to produce antibodies of the present invention is host cell derived from SP2/O.

A skilled person can also use another Expression System which comprises of fusion cells where one host cell containing nucleotide sequences expressing amino acid sequences of monoclonal antibodies of the present invention is fused with another host cell to develop the expression system. Method for producing an antibodies capable of binding and neutralizing rabies or rabies related viruses of the present invention comprising mainly i) culturing the host cell expressing monoclonal antibody capable of binding and neutralizing rabies or rabies related viruses; and ii) isolating and recovering monoclonal antibody capable of binding and neutralizing rabies or rabies related viruses expressed in the said host cell.

Culturing the host cell expressing monoclonal antibody capable of binding and neutralizing rabies or rabies related viruses includes preferably production of monoclonal antibody by Sp2/0 cell culture process in suitable media which is known in the art. Expression of the monoclonal antibody occurs in a constitutive manner and secreted by the cells in soluble form.

Isolating and recovering monoclonal antibody capable of binding and neutralizing rabies or rabies related viruses expressed in the said host cell include mainly cell clarification, affinity chromatography in combination with other suitable chromatography techniques and/or filtration techniques to prepare a purified bulk drug substance of monoclonal antibody of interest. The purified monoclonal antibody or its cocktail preparation is tested for neutralization of various lyssaviruses strains.

The present invention also provides a stable pharmaceutical composition comprising a cocktail of two monoclonal antibodies and a pharmaceutically acceptable carrier or excipient. Preferred excipients are buffer, salt and surfactant. Suitable excipients to formulate and to prepare final bulk drug product can be selected by the skilled person.

The pharmaceutical composition according to the current invention includes therapeutic potency of monoclonal antibody or a cocktail of two monoclonal antibodies. The said therapeutic potency is in the range from 1 IU/mL to 5000 IU/mL, preferably 100 IU/mL to 3000 IU/mL. The therapeutic potency according to present disclosure include each integer and non-integer number between the mentioned ranges i.e. 1 IU/mL to 5000 IU/mL. For Example, the said range includes 10 IU/mL, 25 IU/mL, 50 IU/mL, 100 IU/mL, 150 IU/mL, 200 IU/mL, 300 IU/mL, 400 IU/mL, 500 IU/mL, 600 IU/mL, 700 IU/mL, 800 IU/mL, 900 IU/mL, 1000 IU/mL, 1200 IU/mL, 2000 IU/mL, 3000 IU/mL, 4000 IU/mL and 5000 IU/mL. Cocktail of antibodies according to the current invention may include therapeutic potency of each monoclonal antibody in the described therapeutic range. Preferably, cocktail of antibodies includes therapeutic amount, preferably equipotent amount of each monoclonal antibody in the described therapeutic range. The preferred therapeutic potency of antibody according to the present invention is between 100 IU/mL - 3000 IU/mL. In one of the embodiments, preferred therapeutic potency of cocktail according to the present invention is between 400 IU/mL - 1500 IU/mL. More preferably, therapeutic potency of antibody according to the present invention is 150 IU/mL or 300 IU/mL or 600 IU/mL. More preferably, therapeutic potency of cocktail according to the present invention is 300 IU/mL or 600 IU/mL or 1200 IU/mL. The preventive and therapeutic composition of the present invention is sterile and stable under the conditions of manufacture and storage. It can be in solution and an appropriate pharmaceutically acceptable excipient can be added and/or mixed before or at the time of delivery to provide a unit dosage injectable form. Alternatively, the composition of the present invention can be in lyophilized form which can be reconstituted in an appropriate pharmaceutically acceptable excipient before or at the time of delivery. In the case of sterile powders for the preparation of sterile injectable solutions, preferred preparation method is freeze-drying. The "lyophilized or freeze-drying composition" is a dosage form which is prepared by lyophilization or freeze drying process. The lyophilization was performed with conventional lyophilization technique known in the literatures involving steps such as freezing, annealing, primary drying and secondary drying.

A composition of cocktail of two monoclonal antibodies according to the present invention can be used for treatment and prevention of rabies, preferably for post-bite prophylaxis. The cocktail according to the present invention is studied to check rabies virus neutralization activity. The potency of the rabies or rabies related viruses neutralizing antibodies is determined by RFFIT in comparison with an 'Anti-rabies Immunoglobulin, Human WHO International Standard' (RAI) or a validated internal reference standard against the international standard. RFFIT assay is useful for the determination of antibodies against the rabies or rabies related viruses in both early phase (passive immunization) and the late phase (active immunization).

The routes of administration of the preventive and therapeutic composition of the present invention can be divided into oral and parenteral routes. The preferred route of administration is infiltration in to and around the site of exposure. Optionally, remaining volume is injected intramuscularly at site(s) distant from the wound site, but is not limited thereto.

The cocktail of two monoclonal antibodies was found to have the ability to neutralize various rabies or rabies related viruses and thus is useful for the treatment and prevention of rabies in the patients and animals infected with rabies or rabies related viruses. Preferably, the cocktail of two monoclonal antibodies can be administered directly in the surrounding area of the exposure upon bite by a rabid animal. Optionally, remaining volume was administered in the different parts of the body intramuscularly.

In one of the embodiments, dose of the preventive and therapeutic cocktail according to the present invention is selected from 1 IU/Kg to 100 IU/Kg. Preferably, dose of the preventive and therapeutic cocktail according to the present invention is selected from 10 IU/Kg to 80 IU/Kg. More preferably, dose of the preventive and therapeutic cocktail according to the present invention is 40 IU/Kg.

The dose regime of the preventive and therapeutic antibodies of the present invention include an administration of antibody of the current invention in conjunction with anti-rabies vaccine. Anti-rabies vaccine according to the present invention can be anti-rabies vaccines available in market or under clinical development. For example, anti-rabies vaccine according to the present invention can be selected from Imovax, Rabavert, Rabipur, Rabivax, Vaxirab N, Abhayrab, Indirab, Verorab, SPEEDA, etc. These are the brand names of the anti-rabies vaccines available in the market. In a preferred embodiment, antibody of the present invention is administered without delay as an emergency procedure. It can also be injected up to 7 days after administration of first vaccine dose. In a preferred embodiment, the cocktail of the present invention can be used for the PEP of exposure with rabies and rabies-related viruses. More preferably, the cocktail of the present invention is used in conjunction with anti-rabies vaccine for the PEP of exposure with rabies and rabies-related viruses.

Analytical methods used in the present invention:

Rapid fluorescent focus inhibition test (RFFIT) for the potency and RVNA titer determination.

The RFFIT assay was carried out in 96-well tissue culture plates. A fixed dose of CVS-11 was incubated with each of the 1:2 serial dilutions of the test and the reference materials. After 90 minutes of such incubation, the test and reference materials along with the CVS-11 virus were added over BHK-21 cells and incubated for a further 22 ± 2 hours

in a 5% $CO_2$ incubator at 37°C. On completion of the incubation period, the cell monolayer was washed and fixed with 80% acetone. The presence of non-neutralized virus at each serial dilution is detected by identifying cells infected by residual virus using a FITC conjugated antibody against the rabies virus nucleoprotein and counting the fluorescing infected cells under a fluorescent microscope. The RVNA titre was calculated as shown below:

Calculation of RVNA Titer

$$\text{Difference of logarithm} = \frac{50\% - \text{Infectivity next below } 50\%}{\text{Infectivity next above } 50\% - \text{Infectivity next below } 50\%}$$

$$\text{Log (reciprocal of 50\% endpoint dilution)} = \text{Log (reciprocal of starting point dilution)} + \text{Difference of logarithm}$$

Endpoint Titer = Antilog of Log (reciprocal of 50% endpoint dilution)

$$\text{RVNA Titer (IU/mL)} = \frac{\text{Endpoint titer of sample}}{\text{Endpoint titer of Ref. Std.}} \times 2 \text{ IU/mL (Reference Standard)}$$

Here, the present invention is illustrated with the following non-limiting examples which should not be interpreted as limiting the scope of the invention in any way.

**Examples**

**Example 1: Manufacturing of monoclonal antibody mAb A active drug substance**

Drug substance manufacturing process of mAb A monoclonal antibody involves -

- Upstream Sp2/0 cell culture process and
- Downstream purification process.

Upstream culture process to produce mAb A monoclonal antibody

mAb A monoclonal antibody was produced by host cell of the expression system in Sp2/0 cell culture process in the presence of animal origin free media and feed components. Serum components containing media can also be used to produce the said antibody. Each batch production in upstream was initiated with the revival of a master cell bank (MCB) or Working Cell Bank (WCB) vial content of Sp2/0 cell harboring the mAb A polynucleotide sequence. Following revival of the cell bank vial, a series of seed development steps was performed to generate the final inoculum with adequate number of cells and inoculated into the production bioreactor. The cell culture process in bioreactor was conducted in a well-controlled manner with various on-line and off-line control parameters to express the mAb A monoclonal antibody, in a constitutive manner. Protein was secreted by the cells in soluble form.

Multiple batches of a composition comprising the monoclonal antibody were produced. An inoculum of the mAb A was cultured in 200L bioreactors containing a liquid cell culture medium. The mature antibody encoded by these nucleic acids is mAb A and comprises the heavy chain amino acid sequence set forth in SEQ ID NO:9 and the light chain amino acid sequence shown in SEQ ID NO: 10. Cell culture was performed in a controlled environment by maintaining pH 7.0 ± 0.3 using CO2 gas and /or sodium bicarbonate, as and when required. The dissolved oxygen concentration was maintained at 15 ± 5% saturation with sparging of air and/or oxygen gas and by controlling agitation speed in the bioreactor. Bioreactors were operated at a temperature of about 36.0° C to about 37.0° C.

Growth media contains following components:

| Components | Concentration per liter |
|---|---|
| BD CELLTM MAB ACF | 12.860 gm |

Cells were grown under the above-mentioned conditions for two days from day 2, feeding was initiated and continued until the end of batch. Following media components were fed to the cell culture medium as common feed -

| Components | Concentration per liter |
|---|---|
| L-Glutamine | 2 mM daily from Day 2 onwards until the day before harvest |

(continued)

| Components | Concentration per liter |
|---|---|
| Concentrated growth media | 2% Feed (v / v) with respect to the initial working volume in bioreactor from Day 3 onwards until the day before harvest |

The culture duration was about 7 to 9 days. The in vitro cell age (culture days from initial thaw of master cell bank to harvest) was 26 days or less. After this, the clarified cell culture supernatant was harvested by centrifugation and depth filtration of the cell culture medium. This clarified supernatant was then reconditioned to match substantially to the next purification column equilibration conditions. Supernatant was then subjected to protein a chromatography and impurities were allowed to flow off this chromatography column.

Downstream purification process to obtain the mAb A monoclonal antibody drug substance

Purification process of mAb A monoclonal antibody was carried out with several unit operations comprising cell clarification, reconditioning, chromatography, viral inactivation, virus clearance and membrane ultrafiltration-diafiltration. Purification by chromatography involved various interaction chromatography techniques, like affinity chromatography and other suitable chromatography and filtration techniques. Viral inactivation was conducted at low pH condition for a certain period of time and virus clearance was performed by nano-filtration. Reconditioning and/or buffer exchange was carried out by conventional membrane ultrafiltration-diafiltration.

At the end of purification process, the purified mAb A monoclonal antibody was filtered through a 0.2 $\mu$m filter, aseptically, and stored under frozen condition in suitable container-closure system. The entire purification process was monitored and controlled with appropriate physical and physicochemical parameters related to mAb A monoclonal antibody and its purification process. When assessed by analytical high-performance size exclusion chromatography, the mAb A drug substance was found to show greater than 99 % purity.

## Example 2: Manufacturing of mAb B monoclonal antibody active drug substance

Drug substance manufacturing process of mAb B monoclonal antibody involves -

- Upstream Sp2/0 cell culture process and
- Downstream purification process.

Upstream culture process to produce mAb B monoclonal antibody

mAb B monoclonal antibody was produced by host cell of the expression system Sp2/0 in cell culture process in the presence of animal origin free media and feed components. Serum components containing media can also be used to produce the said antibody. Each batch production in upstream was initiated with the revival of a master cell bank (MCB) or Working Cell Bank (WCB) vial content of Sp2/0 cell harboring the polynucleotide sequence of mAb B. Following revival of the cell bank vial, a series of seed development steps was performed to generate the final inoculum with adequate number of cells and inoculated into the production bioreactor. The cell culture process in bioreactor was conducted in a well-controlled manner with various on-line and off-line control parameters to express the mAb B monoclonal antibody, in a constitutive manner. Protein was secreted by the cells in soluble form.

Multiple batches of a composition comprising the monoclonal antibody were produced. An inoculum of the mAb B was cultured in 200L bioreactors containing a liquid cell culture medium. The mature antibody encoded by these nucleic acids is mAb B and comprises the heavy chain amino acid sequence set forth in SEQ ID NO: 11 and the light chain amino acid sequence shown in SEQ ID NO: 12. Cell culture was performed in a controlled environment by maintaining pH 7.0 $\pm$ 0.3 using CO2 gas and /or sodium bicarbonate, as and when required. The dissolved oxygen concentration was maintained at 30 $\pm$ 10% saturation with sparging of air and/or oxygen gas and by controlling agitation speed in the bioreactor. Bioreactors were operated at a temperature of about 36.0° C to about 37.0° C.

Growth media contains following components:

| Components | Concentration per liter |
|---|---|
| BD CellTM MAb ACF | 12.860 gm |

Cells were grown under the above-mentioned conditions for two days. From day 2, feeding was initiated and continued until the end of batch. Following media components were fed to the cell culture medium as common feed -

Growth media contained following components:

| Components | Concentration per liter |
|---|---|
| L-Glutamine | 2 mM daily from Day 2 onwards until the day before harvest |
| Feed media Cell Boost 7a | 1% Feed (v / v) with respect to the initial working volume in bioreactor from Day 3 onwards until the day before harvest |
| Feed media Cell Boost 7b | 0.3% Feed (v / v) with respect to the initial working volume in bioreactor from Day 3 onwards until the day before harvest |

The culture duration was about 5 to 7 days. The *in vitro* cell age (culture days from initial thaw of master cell bank to harvest) was 33 days or less. After this, the clarified cell culture supernatant was harvested by centrifugation and depth filtration of the cell culture medium. This clarified supernatant was then reconditioned to match substantially to the next purification column equilibration conditions. Supernatant was then subjected to protein A chromatography and impurities were allowed to flow off this chromatography column.

Downstream purification process to obtain the mAb B monoclonal antibody drug substance

Purification process of mAb B monoclonal antibody was carried out with several unit operations comprising cell clarification, reconditioning, chromatography, viral inactivation, virus clearance and membrane ultrafiltration-diafiltration. Purification by chromatography involved various interaction chromatography techniques, like affinity chromatography and other suitable chromatography and filtration techniques. Viral inactivation was conducted at low pH condition for a certain period of time and virus clearance was performed by nano-filtration. Reconditioning and/or buffer exchange was carried out by conventional membrane ultrafiltration-diafiltration.

At the end of purification process, the purified mAb B monoclonal antibody was filtered through a 0.2 $\mu$m filter, aseptically, and stored under frozen condition in suitable container-closure system. The entire purification process was monitored and controlled with appropriate physical and physicochemical parameters related to mAb B monoclonal antibody and its purification process. When assessed by analytical high-performance size exclusion chromatography, the mAb B drug substance was found to show greater than 99 % purity.

**Example 3: Preparation of antibody cocktail comprising equipotent antibodies**

To prepare an antibody cocktail comprising equipotent amount of two antibodies, a potency value in IU/mg was assigned to the purified drug substance of mAbs A and mAbs B by undertaking a RFFIT test. Individual drug substances mAbs A and mAbs B were diluted based on the concentration (mg/mL) to arrive at a similar potency value in IU/mL by citrate buffer of pH 6.0 containing sodium chloride and polysorbate. To prepare an antibody cocktail comprising equipotent antibodies, both the diluted drug substance of mAbs A and mAbs B were suitably mixed.

**Example 4 Manufacturing of monoclonal antibody cocktail**

Antibody cocktail was prepared by mixing equipotent amounts of the mAb A and mAb B monoclonal antibody drug substances in the presence of citrate buffer of pH 6.0 containing sodium chloride and polysorbate. Compounding was performed in a step-wise manner in a suitable vessel. pH of the solution at different stages of compounding was controlled and mixing of solution was conducted thoroughly. Upon completion of compounding, an aliquot of the formulated bulk was withdrawn and analyzed for pH and osmolality. For bioburden reduction and sterile filtration of the final formulated bulk, 0.2 $\mu$m sterilizing grade filters were used, prior to filling in to the glass vials or syringes. Filter integrity was tested before (pre) and after (post) filtration of the bulk. Filling was performed by using a pump and fill volume was checked by weight throughout the entire filling process. On-line stoppering of the filled-in primary containers was conducted, simultaneously, under sterile conditions. After stoppering, flip-off sealing was carried out. The final product thus prepared provides a stable pharmaceutical composition which was found to remain stable for at least 36 months under Real-Time temperature storage conditions in the specified container-closure system.

**Example 5: *In vitro* studies of mAb A and mAb B**

*In-vitro* cross-reactivity of the selected mAbs candidates was measured on a selection of RABV and rabies-related viruses isolated from specific host species and geographical areas in various sets of experiments. Primarily, RABV (genotype 1) of dogs from Asia (India, Philippines, and Thailand etc.), Africa (North Africa, sub-Saharan Africa, etc.) and the New World were included. Also, RABV (genotype 1) from mongoose(s) from South Africa were included. Furthermore, the neutralization capability of the mAbs (mAb A and mAb B) against rabies related viruses, such as EBLV-1, EBLV -2, (genotype 5, 6) ABLV (genotype 7), Duvenhage virus (genotype 4) isolated species including Irkut virus, Khujand and Aravan were tested.

Monolayers of NA cells were infected with selected RABV and rabies-related virus strains at a multiplicity of infectivity of 0.1 for 1 h at 37°C in 5% $CO_2$. The virus inoculum was then removed, fresh medium was added to the cells, and cells were subsequently incubated for 72 h at 37°C in 0.5% $CO_2$. Subsequently, the culture supernatants were collected and titrated on BHK 21 cells. Up to three passages were conducted to obtain sufficient virus titres. Viruses were stored at -80°C until further use.

Rapid fluorescent focus inhibition test (RFFIT) or fluorescent antibody virus neutralisation test (FAVN) assays were performed on BHK 21 cells using a constant virus and varying mAbs method approach. Each mAbs (purified or supernatant) was serially diluted (10.0 - 0.03 IU/ml) and incubated with $10^4$ FFU/ml of the RABV and rabies-related viruses to be tested. To determine the neutralizing potency of each mAbs, 100% reduction of the test virus by mAb A and mAb B was compared to the 100% reduction of the same test virus by an international standard rabies immunoglobulin (SRIG, 2nd human rabies immunoglobulin preparation, National Institute for Biological Standards and Control, Potters Bar, UK). SRIG, used herein is a human derived rabies immunoglobulin which can also be mentioned as HRIG. The test was read after 24 hours of incubation.

**5.1 *In vitro* study of virus neutralization by the selected mAbs**

Table: 1 Neutralisation pattern of Mab A and SRIG using 17 RABV and rabies related viruses. Closed (grey) boxes represent presence of viable virus

| Virus strain | MAb A IU/ml | | | | | | | | | SRIG IU/ml | | | | | | | | |
|---|---|---|---|---|---|---|---|---|---|---|---|---|---|---|---|---|---|---|
| | 10 | 5 | 2 | 1 | 0.5 | 0.25 | 0.125 | 0.0063 | 0.03 | 10 | 5 | 2 | 1 | 0.5 | 0.25 | 0.125 | 0.0063 | 0.03 |
| CVS 11 | | | | | | | | | | | | | | | | | | |
| SAD B19 | | | | | | | | | | | | | | | | | | |

| Virus strain |
|---|
| PV |
| Kelev |
| European Fox |
| Dog Turkey |
| Dog Ethiopia |
| Dog India |
| Dog Mexico |
| Wolf Sarajevo |
| Bobcat-USA |
| EBLV 1 |
| EBLV 2 |
| East European fox |
| Polar fox |
| Dog Azerbaijan |
| Dog Nepal |

# closed boxes represent presence of viable virus

Table:2 Neutralisation pattern of Mab B and SRIG using 17 RABV and rabies related viruses. Closed (grey) boxes represent presence of viable virus

| Virus strain | MAb B IU/ml | | | | | | | | | SRIG IU/ml | | | | | | | | |
|---|---|---|---|---|---|---|---|---|---|---|---|---|---|---|---|---|---|---|
| | 10 | 5 | 2 | 1 | 0.5 | 0.25 | 0.125 | 0.063 | 0.03 | 10 | 5 | 2 | 1 | 0.5 | 0.25 | 0.125 | 0.063 | 0.03 |
| CVS 11 | | | | | | | | | | | | | | | | | | |
| SAD B19 | | | | | | | | | | | | | | | | | | |
| PV | | | | | | | | | | | | | | | | | | |
| Kelev | | | | | | | | | | | | | | | | | | |
| European Fox | | | | | | | | | | | | | | | | | | |
| Dog Turkey | | | | | | | | | | | | | | | | | | |
| Dog Ethiopia | | | | | | | | | | | | | | | | | | |
| Dog India | | | | | | | | | | | | | | | | | | |
| Dog Mexico | | | | | | | | | | | | | | | | | | |
| Wolf Sarajevo | | | | | | | | | | | | | | | | | | |
| Bobcat-USA | | | | | | | | | | | | | | | | | | |

| | | | | | | | | | | | | | | | | | | |
|---|---|---|---|---|---|---|---|---|---|---|---|---|---|---|---|---|---|---|
| EBLV 1 | ■ | ■ | ■ | ■ | ■ | ■ | | | | | | ■ | ■ | ■ | ■ | ■ | ■ | ■ |
| EBLV 2 | | | | | | ■ | ■ | | | ■ | ■ | ■ | ■ | ■ | ■ | ■ | ■ | ■ |
| East European fox | | | | | | ■ | ■ | | | | | ■ | ■ | ■ | ■ | ■ | ■ | ■ |
| Polar fox | | | ■ | ■ | | | | | | | | | | ■ | ■ | ■ | ■ | ■ |
| Dog Azerbaijan | | | | ■ | ■ | ■ | | | | | | | ■ | ■ | ■ | ■ | ■ | ■ |
| Dog Nepal | | | | | ■ | ■ | | | | | ■ | ■ | ■ | ■ | ■ | ■ | ■ | ■ |

# closed boxes represent presence of viable virus

Both the mAbs were tested as a re-suspended purified antibody starting from a potency of 10 IU/mL up to a dilution of 0.03 IU/mL. Additionally, the antibodies were found to be complementary in their neutralization potential. The test demonstrated that the antibodies selected was comparable to SRIG with respect to the breadth of virus neutralization.

**5.2 *In vitro* study of virus neutralization by the selected mAbs**

In the *in vitro* testing assays, seven different RABV (2 laboratory strains and 5 RABV field strains) were used to determine the neutralisation potency of each mAb. Furthermore, 4 different rabies-related viruses representing genotypes 4, 5 and 6 as well as 4 isolates of bat lyssaviruses from Central Asia and East Siberia [Australian bat virus (ABV), Aravan virus (ARAV), Khujand virus (KHUV) and Irkut virus (IRKV)] were included in the virus panel.

Table: 3 Neutralisation pattern of SRIG, mAb A and B using RABV and rabies related viruses

| Virus strain \ IU/ml | SRIG | | | | mAb A | | | | mAb B | | | |
|---|---|---|---|---|---|---|---|---|---|---|---|---|
| | 1:5 | 1:25 | 1:12 | 1:62 | 1:5 | 1:25 | 1:12 | 1:62 | 1:5 | 1:25 | 1:12 | 1:62 |
| CVS-11 | | | | ■ | | | | ■ | | | | ■ |
| Raccoon, SE US | | | ■ | ■ | | | | ■ | | | | ■ |
| Gray fox, TX | | | ■ | | | | | ■ | | | | |
| Arctic fox, Alaska | | | ■ | | | | | ■ | | | | ■ |
| Skunk, SC US | | | ■ | | | | | ■ | | ■ | ■ | |
| Skunk, CA | | | ■ | ■ | ■ | ■ | ■ | ■ | | | | |
| ABV | | | ■ | | | | | ■ | | | | ■ |
| CVS-11 | | | ■ | | | | | ■ | | | | |
| Duvenhage | | | ■ | ■ | | | | ■ | | ■ | ■ | |
| Irkut | | | ■ | | | | | ■ | | | | |
| Aravan | | | ■ | ■ | | | | ■ | | | | |
| Khujand | | | ■ | ■ | | | | ■ | | | | |
| EBLV-1 | | | ■ | ■ | | | | ■ | | ■ | ■ | |
| EBLV-2 | | | ■ | ■ | | | | ■ | | | | |

# closed boxes represent presence of viable virus

There is a broad spectrum *in-vitro* cross-reactivity of the candidate mAbs against rabies and some other lyssaviruses. All the rabies and rabies related viruses were neutralized by either of the monoclonal antibodies. Additionally, the antibodies were found to be complementary in their neutralization potential. The test demonstrated that the antibodies selected were comparable to SRIG with respect to the breadth of virus neutralization. The data demonstrated similarity between the antibodies and SRIG in their ability to neutralize the different rabies and RABV like lyssaviruses.

**5.3 *In vitro* study of virus neutralization by cocktail of mAb A and mAb B, HRIG and ERIG**

Experiment was conducted against street rabies virus isolates and the CVS strain.

Table 4: Neutralisation pattern of mAb cocktail, HRIG and ERIG

| Virus strain | Source of isolates | Virus Neutralization | | | | | |
|---|---|---|---|---|---|---|---|
| | | mAb cocktail (mAb A and mAb B) | | HRIG | | ERIG | |
| | | 10 IU | 100 IU | 10 IU | 100 IU | 10 IU | 100 IU |
| CVS | - | 100 % | 100 % | 100 % | 100 % | 100 % | 100 % |
| SV1 | Dog | 100 % | 100 % | 100 % | 100 % | 100 % | 100 % |
| SV2 | Canine | 100 % | 100 % | 100 % | 100 % | 100 % | 100 % |
| SV3 | Human | 100 % | 100 % | 100 % | 100 % | 100 % | 100 % |
| SV4 | Dog | 100 % | 100 % | 100 % | 100 % | 100 % | 100 % |
| SV5 | Human | 100 % | 100 % | 100 % | 100 % | 100 % | 100 % |
| SV6 | Human | 100 % | 100 % | 100 % | 100 % | 100 % | 100 % |
| SV7 | Dog | 100 % | 100 % | 100 % | 100 % | 100 % | 100 % |
| SV8 | Dog | 100 % | 100 % | 100 % | 100 % | 100 % | 100 % |
| SV9 | Dog | 100 % | 100 % | 100 % | 100 % | 100 % | 100 % |
| SV10 | Bovine | 100 % | 100 % | 100 % | 100 % | 100 % | 100 % |

The cocktail of mAbs A and B was found to neutralize all the isolates similar to the neutralization by HRIG (Human rabies immunoglobulin preparation) and ERIG (Equine rabies immunoglobulin preparation).

Example 6: *In vivo* **potency studies of mAb cocktail**

6.1 *In vivo* **potency study of mAb cocktail, ERIG and HRIG**

In this study, mice was used as animal model. Virus challenged animals were divided in 5 study groups: 1) Control group injected with normal saline; 2) injected with 0.2 IU/mice mAb cocktail; 3) injected with 0.4 IU/mice mAb cocktail; 4) injected with 0.2 IU/mice HRIG; 5) injected with 0.4 IU/mice ERIG.

In this study, CVS virus and 10 strains of street virus were used to challenge the animals. The mAb cocktail was administered at two doses 0.2 IU/mice and 0.4 IU/mice in order to compare against a similar dose of HRIG and ERIG. The dilution of the mAb to be tested was calculated on the basis of recommended doses of HRIG (20 IU/Kg) and ERIG (40 IU/Kg). Each mice was injected with HRIG at 0.2 IU/mice in group 4 and ERIG at 0.4 IU/mice in group 5. mAb cocktail was tested on both the doses 0.2 IU/mice and 0.4 IU/mice in group 2 and in group 3, respectively .

Each group having 8 mice were challenged with 100 $LD_{50}$ of each mentioned virus. After 1 hour of virus challenge, control group received 100 $\mu$L of normal saline inoculated on the same site as the virus inoculation. Rest of the four groups received one of the dilution of mAbs infiltered in the same area where virus was inoculated. The mice were kept under observation for 1 month.

Table: 7 Animal study of mAb cocktail, ERIG and HRIG in virus challenged mice

| Experiment No. | Animal model | Virus strain | % survival | | | | |
| | | | Placebo | mAb cocktail (mAb A + mAb B) | | HRIG 0.2 IU/ mice | ERIG 0.4 IU/ mice |
| | | | | 0.2 IU/ mice | 0.4 IU/ mice | | |
| 1 | Mice | CVS | 0 | 100 | 100 | 100 | 100 |
| | | SV1 | 10 | 100 | 100 | 100 | 100 |
| | | SV2 | 10 | 100 | 100 | 100 | 100 |
| | | SV3 | 0 | 100 | 100 | 100 | 100 |
| | | SV4 | 20 | 100 | 100 | 100 | 100 |
| | | SV5 | 0 | 100 | 100 | 100 | 100 |
| | | SV6 | 0 | 100 | 100 | 100 | 100 |
| | | SV7 | 10 | 100 | 100 | 100 | 100 |
| | | SV8 | 10 | 100 | 100 | 100 | 100 |
| | | SV9 | 0 | 100 | 100 | 100 | 100 |
| | | SV10 | 0 | 100 | 100 | 100 | 100 |

Results provided in table 7 shows that survival rate of 100% was observed in study group treated with anti-rabies monoclonal antibody (HRIG, ERIG and mAb cocktail). While in the control group challenged with CVS, 100% mortality was observed.

It also proves that mAb cocktail of the present invention is as potent as currently approved antibodies (HRIG and ERIG).

### 6.2 In vivo potency study of mAb cocktail and HRIG

In order to establish PK/PD correlation for appropriate design of clinical study in human, the *in-vivo* efficacy study in rabies virus (CVS-11) challenged hamsters was performed.

The efficacy determination was based on the number of hamsters surviving a lethal dose of rabies virus, upon treatment with the equal dose of the test sample mAb cocktail and the standard HRIG (Kamrab, Kamada Ltd., Beit-Kama, Israel).

Study animals were randomized based on their body weight and divided in different treatment groups. Virus challenged animals were divided (10 animals in each group) into the following three groups: 1) a group injected with CVS-11 virus alone; 2) a group injected with CVS-11 virus and Anti rabies mAbs cocktail individually; 3) ) a group injected with CVS-11 virus and standard HRIG (Kamrab, Kamada Ltd., Beit-Kama, Israel).

Rabies virus preparation with a known $LD_{50}$ titer was suitably diluted and 200 μL administered into the right gastrocnemius muscle of each hamster. The formulated solution of the Anti-rabies mAbs cocktail and the standard Kamrab were administered at 6 hours into defined groups at the same site of virus injection in fed conditions. The dose level selected was 15 IU/hamster to minimize the dose volume at injection site (~100 μL). Hamsters were observed daily for any clinical signs of rabies such as paralysis or death. Upon observation of any signs of rabies virus infection, the animals were euthanized by carbon dioxide asphyxiation. % survival was checked after 30 days from virus challenge. The results of the study are shown in table 8.

Table: 8 Animal study of mAb cocktail and HRIG dosing without vaccine after 6 hours of CVS-11 virus challenge

| Experiment No. | Animal model | Challenge virus | Group (Drug administered 6 hours after virus challenge) | Dose (i.m. route) | Survival | % survival |
|---|---|---|---|---|---|---|
| 1 | Hamsters | CVS-11 (i.m. route) | Control | 100μL | 4/10 | 40% |
| | | | HRIG (Kamrab, Kamada Ltd., Beit-Kama, Israel) | 100μL (15IU) | 5/10 | 50% |
| | | | Test (mAb cocktail) | 100μL (15IU) | 9/10 | 90% |

The *in vivo* efficacy study of mAb cocktail in comparison with HRIG (Kamrab, Kamada Ltd., Beit-Kama, Israel) was undertaken to demonstrate the virus neutralization potential of mAb cocktail in rabies virus challenged hamsters. In this study, where animals were treated 6 hours after virus challenge showed 90% survival by mAb cocktail of the present invention. It is better than 50% survival by HRIG (Kamrab, Kamada Ltd., Beit-Kama)

**Example 7: Phase III clinical study of mAb cocktail**

The efficacy of a cocktail comprising mAb A and mAb B was studied in a randomized, multi-centric, open-label, comparator-controlled, phase III human clinical study to evaluate and compare the efficacy and safety of the cocktail with an HRIG in a PEP setting, where, both were administered in conjunction with an anti-rabies vaccine.

The study comprised of 308 subjects with WHO Category III exposure(s) from suspected rabid animals that were divided equally into two arms. In one arm, subjects were given mAb cocktail (40 IU/kg) in conjunction with an anti-rabies vaccine (VaxiRab N; Cadila Healthcare Ltd. Ahmedabad, India) and in the other arm, an HRIG (Imogam® (20 IU/kg, Sanofi Pasteur SA France) with anti-rabies vaccine.

In both arms the drugs being compared were administered directly in the surrounding area of the wounds from the bite of a rabid animal (single site/multiple sites) and remaining volume was administered in the different parts of the body intramuscularly. Both drugs were administered on day 0 in conjunction with an anti-rabies vaccine as per Essen regimen for rabies vaccination (five intramuscular doses on days 0, 3, 7, 14 and, 28). The study was followed for 84 days for each subject.

**Results:**

Both the drugs, Mab cocktail and HRIG, were found to be at least comparable in terms of RVNA titers as assessed at both the early time-points (days 3 and 7), and late time-points (days 14, 28, 42, and, 84). The study demonstrated that the mAb cocktail like HRIG, provided an unbroken window of protection i.e., without significantly neutralizing the anti-rabies vaccine throughout the study duration. Further, the late time-point titers as observed on days 28, 42 and 84 were higher in the mAb cocktail arm as compared to HRIG, confirming that the murine mAb cocktail neutralized the vaccine (active immunization) lesser than the human-derived polyclonal (HRIG), demonstrating the advantage of murine monoclonal antibodies over human antibodies in the PEP setting.

**Equivalents**

The invention may be embodied in other specific forms without departing from the spirit or essential characteristics thereof. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

**Claims**

1. A cocktail of two monoclonal antibodies comprising one monoclonal antibody having heavy chain and light chain comprising polypeptides set forth in SEQ IDs NO. 9 and 10, respectively and second monoclonal antibody having heavy chain and light chain comprising polypeptides set forth in SEQ IDs NO. 11 and 12, respectively.

**2.** The cocktail of two monoclonal antibodies, as claimed in Claim 1, wherein each antibody is a murine monoclonal antibody.

**3.** A cocktail of two monoclonal antibodies, for use in the post-exposure prophylaxis (PEP) of a subject that has undergone exposure with rabies and rabies-related viruses, wherein the cocktail of two monoclonal antibodies, is as claimed in Claim 1 or Claim 2.

**4.** A combination of a cocktail of two monoclonal antibodies, and an anti-rabies vaccine for use in the post-exposure prophylaxis (PEP) of a subject that has undergone exposure with rabies and rabies-related viruses, wherein the cocktail of two monoclonal antibodies, is as claimed in any one of claims 1-3.

**5.** A composition, such as a lyophilized formulation, comprising the cocktail of two monoclonal antibodies, of any one of claims 1-3, and a pharmaceutically acceptable carrier.

**6.** The composition as claimed in claim 5, wherein i) one of the antibodies of the cocktail of two monoclonal antibodies, is present in the range of 1 IU/mL to 5000 IU/mL; or ii) the cocktail of two monoclonal antibodies is present in the range of 100 IU/mL to 1500 IU/mL.

**7.** The composition as claimed in Claim 6, wherein i) one of the antibodies of the cocktail of two monoclonal antibodies is present in the range of 100 IU/mL to 3000 IU/mL; or ii) the cocktail of two monoclonal antibodies is present in the range of 400 IU/mL to 1500 IU/mL.

**8.** The composition as claimed in Claim 6, wherein i) one of the antibodies of the cocktail of two monoclonal antibodies is present in the range of 150 IU/mL or 300 IU/mL or 600 IU/mL; or ii) the cocktail of two monoclonal antibodies is present in the range of 300 IU/mL or 600 IU/mL or 1200 IU/mL.

**9.** The cocktail of two monoclonal antibodies, for use of claim 3, or claim 4, wherein the cocktail of two monoclonal antibodies, is administered: i) via infiltration in to and around the site of exposure and/or intramuscularly at site(s) distant from the wound site; ii) at a dose of 1 IU/Kg to 100 lU/Kg; and/or iii) up to 7 days after the exposure beginning immediately after the exposure.

**10.** The cocktail of two monoclonal antibodies for use according to Claim 9, wherein the cocktail of two monoclonal antibodies, is administered: i) via infiltration in to and around the site of exposure and/or intramuscularly at site(s) distant from the wound site; ii) at a dose of 10 IU/Kg to 80 lU/Kg; and/or iii) up to 7 days after the exposure beginning immediately after the exposure.

**11.** The cocktail of two monoclonal antibodies for use according to Claim 9, wherein the cocktail of two monoclonal antibodies, is administered: i) via infiltration in to and around the site of exposure and/or intramuscularly at site(s) distant from the wound site; ii) at a dose of 40 lU/Kg; and/or iii) up to 7 days after the exposure beginning immediately after the exposure.

**12.** A kit comprising the cocktail of two monoclonal antibodies, as claimed in any of claims 1-3.

**13.** A cocktail of two monoclonal antibodies, for use in diagnosing or detecting rabies, where the cocktail of two monoclonal antibodies is as claimed in any of claims 1-3.


**Patentansprüche**

**1.** Ein Cocktail von zwei monoklonalen Antikörpern, der Folgendes umfasst: einen monoklonalen Antikörper mit schwerer Kette und leichter Kette, der die in SEQ IDs NO. 9 bzw. 10 dargelegten Polypeptide umfasst, und einen zweiten monoklonalen Antikörper mit schwerer Kette und leichter Kette, der die in SEQ IDs NO. 11 bzw. 12 dargelegten Polypeptide umfasst.

**2.** Cocktail von zwei monoklonalen Antikörpern nach Anspruch 1, wobei jeder Antikörper ein monoklonaler Mausantikörper ist.

**3.** Ein Cocktail von zwei monoklonalen Antikörpern zur Verwendung in der Postexpositionsprophylaxe (PEP) eines Individuums, bei dem eine Exposition gegenüber Tollwut und mit Tollwut verwandten Viren erfolgt ist, wobei der Cocktail von zwei monoklonalen Antikörpern gemäß Anspruch 1 oder Anspruch 2 ist.

4. Eine Kombination eines Cocktails von zwei monoklonalen Antikörpern und eines Antitollwut-Impfstoffs zur Verwendung in der Postexpositionsprophylaxe (PEP) eines Individuums, bei dem eine Exposition gegenüber Tollwut und mit Tollwut verwandten Viren erfolgt ist, wobei der Cocktail von zwei monoklonalen Antikörpern gemäß einem der Ansprüche 1-3 ist.

5. Eine Zusammensetzung, wie etwa eine lyophilisierte Formulierung, die den Cocktail von zwei monoklonalen Antikörpern nach einem der Ansprüche 1-3 und einen pharmazeutisch akzeptablen Träger umfasst.

6. Zusammensetzung nach Anspruch 5, wobei i) einer der Antikörper des Cocktails von zwei monoklonalen Antikörpern in dem Bereich von 1 IU/ml bis 5000 IU/ml vorhanden ist; oder ii) der Cocktail von zwei monoklonalen Antikörpern in dem Bereich von 100 IU/ml bis 1500 IU/ml vorhanden ist.

7. Zusammensetzung nach Anspruch 6, wobei i) einer der Antikörper des Cocktails von zwei monoklonalen Antikörpern in dem Bereich von 100 IU/ml bis 3000 IU/ml vorhanden ist; oder ii) der Cocktail von zwei monoklonalen Antikörpern in dem Bereich von 400 IU/ml bis 1500 IU/ml vorhanden ist.

8. Zusammensetzung nach Anspruch 6, wobei i) einer der Antikörper des Cocktails von zwei monoklonalen Antikörpern in dem Bereich von 150 IU/ml oder 300 IU/ml oder 600 IU/ml vorhanden ist; oder ii) der Cocktail von zwei monoklonalen Antikörpern in dem Bereich von 300 IU/ml oder 600 IU/ml oder 1200 IU/ml vorhanden ist.

9. Cocktail von zwei monoklonalen Antikörpern zur Verwendung nach Anspruch 3 oder Anspruch 4, wobei der Cocktail von zwei monoklonalen Antikörpern wie folgt verabreicht wird: i) durch Infiltration in den und um den Ort der Exposition und/oder intramuskulär an (einem) von dem Wundort entfernten Ort(en); ii) mit einer Dosis von 1 IU/kg bis 100 IU/kg; und/oder iii) bis zu 7 Tage nach der Exposition, beginnend unmittelbar nach der Exposition.

10. Cocktail von zwei monoklonalen Antikörpern zur Verwendung nach Anspruch 9, wobei der Cocktail von zwei monoklonalen Antikörpern wie folgt verabreicht wird: i) durch Infiltration in den und um den Ort der Exposition und/oder intramuskulär an (einem) von dem Wundort entfernten Ort(en); ii) mit einer Dosis von 10 IU/kg bis 80 IU/kg; und/oder iii) bis zu 7 Tage nach der Exposition, beginnend unmittelbar nach der Exposition.

11. Cocktail von zwei monoklonalen Antikörpern zur Verwendung nach Anspruch 9, wobei der Cocktail von zwei monoklonalen Antikörpern wie folgt verabreicht wird: i) durch Infiltration in den und um den Ort der Exposition und/oder intramuskulär an (einem) von dem Wundort entfernten Ort(en); ii) mit einer Dosis von 40 IU/kg; und/oder iii) bis zu 7 Tage nach der Exposition, beginnend unmittelbar nach der Exposition.

12. Ein Kit, der den Cocktail von zwei monoklonalen Antikörpern nach einem der Ansprüche 1-3 umfasst.

13. Ein Cocktail von zwei monoklonalen Antikörpern zur Verwendung bei der Diagnose oder dem Nachweis von Tollwut, wobei der Cocktail von zwei monoklonalen Antikörpern gemäß einem der Ansprüche 1-3 ist.

**Revendications**

1. Cocktail de deux anticorps monoclonaux comprenant un anticorps monoclonal comportant une chaîne lourde et une chaîne légère comprenant des polypeptides définis dans les SÉQ. ID nos 9 et 10, respectivement, et un deuxième anticorps monoclonal comportant une chaîne lourde et une chaîne légère comprenant des polypeptides définis dans les SÉQ. ID nos 11 et 12, respectivement.

2. Cocktail de deux anticorps monoclonaux selon la revendication 1, dans lequel chaque anticorps est un anticorps monoclonal murin.

3. Cocktail de deux anticorps monoclonaux destiné à une utilisation dans la prophylaxie post-exposition (PPE) d'un sujet qui a été exposé à la rage et à des virus apparentés à la rage, le cocktail de deux anticorps monoclonaux étant selon la revendication 1 ou la revendication 2.

**4.** Combinaison d'un cocktail de deux anticorps monoclonaux et d'un vaccin antirabique destinée à une utilisation dans la prophylaxie post-exposition (PPE) d'un sujet qui a été exposé à la rage et à des virus apparentés à la rage, le cocktail de deux anticorps monoclonaux étant selon l'une quelconque des revendications 1 à 3.

**5.** Composition, telle qu'une formulation lyophilisée, comprenant le cocktail de deux anticorps monoclonaux selon l'une quelconque des revendications 1 à 3 et un support pharmaceutiquement acceptable.

**6.** Composition selon la revendication 5, dans laquelle i) l'un des anticorps du cocktail de deux anticorps monoclonaux est présent dans la plage de 1 UI/ml à 5 000 UI/ml ; ou ii) le cocktail de deux anticorps monoclonaux est présent dans la plage de 100 UI/ml à 1 500 UI/ml.

**7.** Composition selon la revendication 6, dans laquelle i) l'un des anticorps du cocktail de deux anticorps monoclonaux est présent dans la plage de 100 UI/ml à 3 000 UI/ml ; ou ii) le cocktail de deux anticorps monoclonaux est présent dans la plage de 400 UI/ml à 1 500 UI/ml.

**8.** Composition selon la revendication 6, dans laquelle i) l'un des anticorps du cocktail de deux anticorps monoclonaux est présent dans la plage de 150 UI/ml ou 300 UI/ml ou 600 UI/ml ; ou ii) le cocktail de deux anticorps monoclonaux est présent dans la plage de 300 UI/ml ou 600 UI/ml ou 1 200 UI/ml.

**9.** Cocktail de deux anticorps monoclonaux destiné à une utilisation selon la revendication 3 ou la revendication 4, le cocktail de deux anticorps monoclonaux étant administré : i) par infiltration dans et autour du site d'exposition et/ou par voie intramusculaire dans un ou plusieurs sites éloignés du site de la plaie ; ii) à une dose de 1 UI/Kg à 100 UI/Kg ; et/ou iii) jusqu'à 7 jours après l'exposition, cette période commençant immédiatement après l'exposition.

**10.** Cocktail de deux anticorps monoclonaux destiné à une utilisation selon la revendication 9, le cocktail de deux anticorps monoclonaux étant administré : i) par infiltration dans et autour du site d'exposition et/ou par voie intramusculaire dans un ou plusieurs sites éloignés du site de la plaie ; ii) à une dose de 10 UI/Kg à 80 UI/Kg ; et/ou iii) jusqu'à 7 jours après l'exposition, cette période commençant immédiatement après l'exposition.

**11.** Cocktail de deux anticorps monoclonaux destiné à une utilisation selon la revendication 9, le cocktail de deux anticorps monoclonaux étant administré : i) par infiltration dans et autour du site d'exposition et/ou par voie intramusculaire dans un ou plusieurs sites éloignés du site de la plaie ; ii) à une dose de 40 UI/Kg ; et/ou iii) jusqu'à 7 jours après l'exposition, cette période commençant immédiatement après l'exposition.

**12.** Trousse comprenant le cocktail de deux anticorps monoclonaux selon l'une quelconque des revendications 1 à 3.

**13.** Cocktail de deux anticorps monoclonaux destiné à une utilisation pour diagnostiquer ou détecter la rage, le cocktail de deux anticorps monoclonaux étant selon l'une quelconque des revendications 1 à 3.

**Figure 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013048130 A
- WO 2016078761 A

**Non-patent literature cited in the description**

- *The Journal of Infectious Diseases,* 2014, vol. 210, 200-8
- *J, gen. Virol.,* 1983, vol. 64, 843-851
- *J. gen. Virol.,* 1983, vol. 64, 1649-1656
- *Proc. Natl. Acad. Sci. USA.,* November 1984, vol. 81, 7194-7198